# FASCICULE DE BREVET EUROPEEN

(11) **EP 1 382 317 B1**
(45) Date de publication et mention de la délivrance du brevet: **09.02.2005**
(21) Numéro de dépôt: 03102213.0
(22) Date de dépôt: 17.07.2003
(51) Int. Cl.: A61F 5/01

(54) **Orthèse dynamique**
Dynamische Orthese
Dynamic orthesis

(30) Priorité: 19.07.2002 FR 0209248
(43) Date de publication de la demande: 21.01.2004
(73) Titulaire: SNC Aeropneumatiss, 95330 Domont (FR)
(72) Inventeur: Teyssedre, Hervé, 95330 Domont (FR); Lefort, Gérald, 95340 Persan (FR)
(74) Mandataire: Schmit, Christian Norbert Marie

(56) Documents cités:
- DE-A- 3 720 767
- FR-A- 497 992
- US-A- 5 144 943
- US-A- 6 036 665

## Description

La présente invention a pour objet une orthèse dynamique de pied permettant une aide à la marche, voire une rééducation fonctionnelle, pour des patients atteints d'hémiplégie ou de maladies neurologiques voire de sciatique paralysante ou atteint de sciatique poplité externe nerf tibial antérieur (NPE NTA) et d'affections neuromotrices spastiques ou non (périphériques). L'orthèse est également utilisable après l'utilisation d'un plâtre pour rééduquer le mouvement du pied à la marche.

Certaines affections ont pour conséquence que le pied est ballant, la pointe du pied retombe naturellement vers le sol lorsque la jambe n'est plus en appui sur le sol. En pratique, l'articulation de la cheville est molle du fait d'une absence partielle ou totale de motricité musculaire. Elle ne retient pas, ou pas suffisamment, la pointe du pied dans une direction correspondant à une mobilité et à une motricité recherchée. Par exemple, un patient soufrant d'une telle affection doit, en marchant, lever exagérément la jambe dont le pied est ballant pour éviter de trébucher par accrochage de ce pied sur le sol au moment où cette jambe est lancée vers l'avant.

Les orthèses connues sont la plus part du temps des orthèses rigides : il n'y a plus aucune mobilité du pied. Ou bien une mobilité réduite est permise par la dynamique même de certains matériaux (leurs propriétés élastiques). Mais il n'y a pas de système dynamique indépendant généré. Le mouvement de la marche qui en résulte est également une claudication, différente de la claudication non appareillée, et une entrave à la dynamique physiologique de la marche. Une telle orthèse n'est donc guère plus pratique. Dans d'autres solutions, le pied est porté par une semelle elle-même reliée par une tige semi-rigide au mollet postérieur du patient. La rigidité de la tige et de sa liaison provoque une tenue du pied sensiblement perpendiculairement à la jambe, en même temps qu'une certaine élasticité dans le redressement du pied. En pratique, soit des mécanismes compliqués à câbles sont prévus dans ce but, soit une flexion d'une tige est impliquée.

Les problèmes présentés par ces derniers types de solution sont leur complexité et leurs défauts d'adaptabilité à la taille, au poids, des patients à appareiller. Par ailleurs, la mobilité ainsi procurée est très limitée. En particulier de telles orthèses sont plutôt malaisées à utiliser lors de la marche sur un sol accidenté, par exemple pour des promenades en montagne.

Dans l'invention, on a résolu tous ces problèmes de complexité et de mobilité en choisissant un mode d'aide particulièrement simple. Dans son principe, l'orthèse de l'invention comporte un moyen pour repousser le talon lorsque le pied est ballant (ou pour le tirer vers la jambe lorsque le pied est en appui). L'organe qui sert à l'articulation du pied est tout simplement la cheville et en elle les articulations antérieures : sous-astragaliennes, astragalo scaphoïdienne, calcanéo cuboïdienne, articulations du tarse et du métatarse du patient. L'organe qui sert à l'articulation du pied n'est donc pas, au premier chef, un mécanisme d'articulation supplémentaire. Il suffit en quelque sorte d'accrocher le talon et le pied, on verra plus loin les différentes façons de le faire, et de les manipuler à partir du mollet pour provoquer la mobilité et le maintien recherché.

En particulier pour faciliter la manipulation, on peut mettre en place un dispositif mobile, par exemple un cardan, une rotule ou une liaison élastique, à un endroit qui correspondrait à une rotation sagittale, frontale, et transversale du pied. De cette façon, on permet au pied de se porter de travers sur le sol si celui-ci est irrégulier. Cette caractéristique supplémentaire de l'invention pourrait par ailleurs être utilisée indépendamment du moyen de manipuler le talon. Toutefois elle sera de préférence utilisée en combinaison avec ce moyen de manipuler le talon.

Sur le plan pratique, et bien que cela ne soit pas une obligation, l'action de manipuler le talon et le pied est provoquée par la présence d'un vérin, pneumatique, hydropneumatique ou à ressort, voire motorisé électriquement, placé sur l'arrière du mollet et l'arrière du talon. Une orthèse selon le préambule de la revendication 1 est connue du document US-A-5 144 943.

La manipulation du pied ainsi préconisé par l'invention utilise la cohésion de la cheville et la présence des ligaments qui lient entre eux les différents os de cette cheville. On constate que dans la plupart des affections une certaine tonicité de ces ligaments reste toujours présente et autorise le fonctionnement projeté. En particulier, dans l'invention on peut se passer d'une articulation rapportée comportant une partie accrochée au mollet et une partie accrochée au pied du patient.

L'invention a donc pour objet une orthèse dynamique qui comporte d'une part une chaussure habituelle, et d'autre part un bracelet de jambe et une semelle de pied reliés ensemble par une structure, la structure comportant un vérin élastique déporté sur l'arrière de la jambe et du pied, ladite structure étant extensible longitudinalement pour écarter une partie de la semelle du bracelet, et ladite semelle étant une semelle de voûte plantaire pour être introduite dans la chaussure.

L'invention sera mieux comprise à la lecture de la description qui suit et à l'examen des figures qui l'accompagnent. Celles-ci ne sont présentées qu'à titre indicatif et nullement limitatif de l'invention. Les figures montrent :
- Figure 1 : une vue de côté d'un releveur, ou orthèse dynamique selon l'invention, mis en place sur un pied figurativement représenté. Cette figure est basée sur un exemple de fonctionnement avec cardan et vérin hydropneumatique équipé d'un réglage de pression.
- Figure 2 : une représentation en perspective arrière plongeante de l'orthèse dynamique de la figure 1.

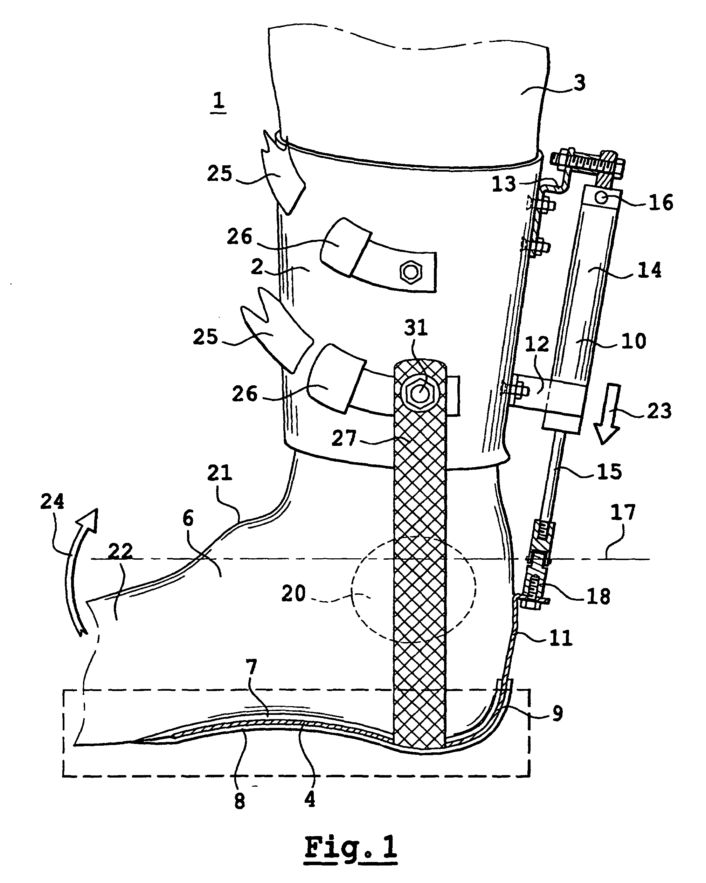

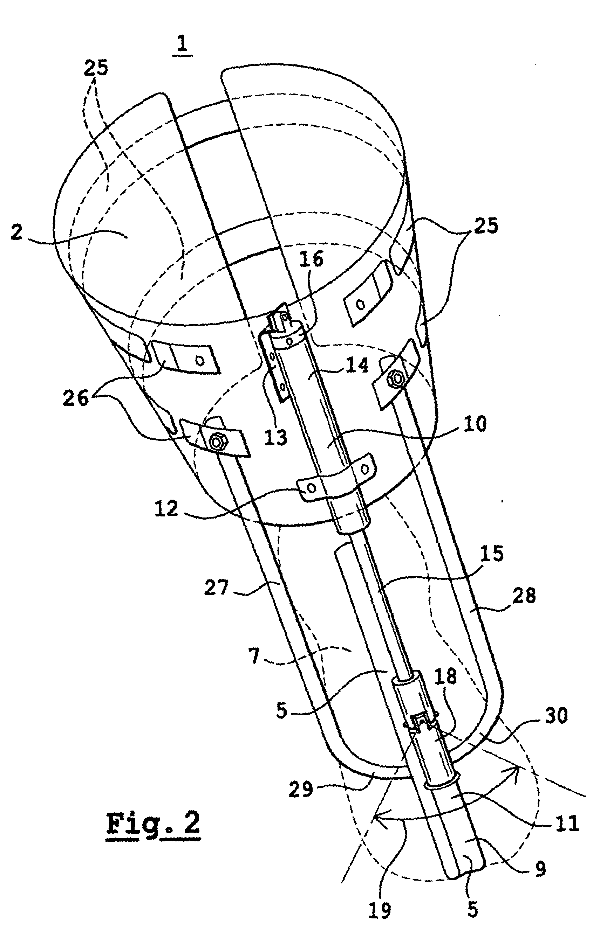

Les figures 1 et 2 montrent une orthèse 1 dynamique selon l'invention comportant un bracelet 2 destiné à être placé sur la jambe 3 d'un patient. Le bracelet 2 peut d'être placé assez près de la cheville, de préférence à la base du mollet de manière à ne pas contraindre le changement de forme de ce dernier au moment des efforts. Le bracelet 2 est par exemple réalisé en matériaux thermoformable ou en cuir. Il pourrait toutefois être réalisé en un matériau élastique extensible, par exemple tissé avec des fils élastiques munis de raidisseurs dans le sens longitudinal.

L'orthèse 1 comporte également une semelle 4 d'un pied 6 du patient. Par semelle 4 on entend essentiellement une tige 5, faite de préférence de matière métallique, figure 2, dont la forme peut être filaire, simplement allongée sous la voûte plantaire du pied 6. Elle est formée selon la voûte plantaire du pied 6.

De préférence, la semelle 4 comportera au moins une coque anatomique 7 surmoulé sur la tige 5 et adaptée de préférence au pied 6 du patient, notamment pour corriger une pathologie statique du pied de ce patient. Par exemple, la correction peut être celle d'un pied un peut trop supinateur pour lequel la coque 7 contribue à la correction. De préférence, la coque 7 sera associée à une coque 8 surmoulée de l'autre côté de la tige 5. Dans un exemple, la coque 7 ou la coque 8 ou les coques 7 et 8 sont en fibre de verre. L'opération de moulage se réalise à partir d'un moule représentant une forme de pied idéale pour le patient concerné, par la mise en place en position intermédiaire des deux coques 7 et 8 (avant polymérisation), puis par la mise en place momentanée du pied 6 du patient sur cet ensemble au moment de, ou préalablement à, la polymérisation. D'autres solutions sont par ailleurs envisageables qui relèvent des techniques des chausseurs ou des podologues.

La semelle 4 et en particulier la tige 5 possèdent une talonnière 9 par laquelle elles remontent du dessous du pied 6, verticalement, orientée en direction de la jambe 3. La talonnière 9 n'est pas indispensable mais permet à l'orthèse de pouvoir être facilement enfilée autour du pied 6, dans une chaussure non représentée. Par ailleurs, l'orthèse de l'invention apporte l'avantage de pouvoir conserver la taille habituelle des chaussures, sauf dans le cas exceptionnel d'une morphologie spécifique.

Le bracelet 2 est relié à la semelle 4, notamment à la talonnière 9, par une structure 10. L'invention, dans un de ses aspects, est essentiellement caractérisée par le fait que la structure 10 comporte un moyen élastique extensible et ou compressible longitudinalement selon le sens de la jambe 3, pour écarter une partie de la semelle, en particulier la talonnière 9, du bracelet 2. Dans l'exemple représenté, la structure élastique extensible et ou compressible longitudinalement comporte un vérin 10, maintenu d'une part sur le bracelet 2 et d'autre part sur la semelle 4, par exemple à l'extrémité 11 de la talonnière 9.

Pour sa fixation sur le bracelet 2, le moyen élastique 10 peut comporter un collier 12 de fixation déportée et un ancrage déporté supérieur 13, tous deux montés sur le bracelet 2 à l'arrière du mollet. Le collier 12 et l'ancrage 13 sont répartis sur le bracelet 2, longitudinalement par rapport à la direction de la jambe 3, de façon à imposer une direction longitudinale au moyen 10, orienté ainsi de la jambe 3 vers le pied 6. Ces deux moyens de fixation pourraient toutefois être remplacés par un seul moyen, du moment que celui-ci assurerait également un maintien longitudinal du moyen élastique 10. Dans certains cas toutefois, notamment lorsqu'il n'y a pas de cardan, un tel double ancrage n'est pas nécessaire mais un seul point de fixation peut être utilisé : l'orientation longitudinale résultant naturellement de la fixation du vérin 10 au bracelet 2 et sur la partie 11 de la semelle 4.

Le moyen 10 élastique extensible et compressible longitudinalement est dans un exemple un vérin pneumatique. Ce vérin pneumatique comporte un corps de vérin 14 attaché au bracelet 2 et une tige de vérin 15 attachée à la semelle 4, en pratique à l'extrémité 11 de la talonnière 9 (ou l'inverse). En fonction des besoins le vérin peut être doublé. Les deux vérins sont dans ce cas automatiquement désaxés et montés de part et d'autre de l'axe du talon. Le réglage de ce vérin est de préférence tel qu'au repos, lorsque le pied 6 est sensiblement perpendiculaire à l'orientation de la jambe 3, se trouve lui-même en semi-compression prêt à relever le pied dès que le patient soulève sa jambe du sol.

Le vérin 10 peut néanmoins être remplacé par un ressort, de préférence un système de ressorts admettant une compression et une extension au delà d'un point d'équilibre correspondant à la position de repos. Ce système de ressorts maintient ainsi un état d'équilibre, sans poussée ou sans traction supérieure à la force musculaire, pour garder une position du pied perpendiculaire à la jambe. En variante, le moyen élastique extensible longitudinalement pourrait être formé par une vis sans fin motorisée par un moteur électrique, et un écrou réglant une hauteur, la commande du moteur étant conditionnée par l'état d'un capteur de la position du pied.

Lorsque le vérin est pneumatique ou hydropneumatique, il possède de préférence une valve 16 pour en régler la pression de manière à adapter l'effort du vérin à la taille du patient à appareiller. Par exemple, si le patient est un adulte d'environ 70 kg, l'effort de poussée procuré par le vérin sera logiquement plus importante que par exemple pour un appareillage destiné à un enfant. Les réglages standards moyens oscillent entre 25 et 50 DaN pour un adulte. Par contre, s'il s'agit de l'appareillage d'un enfant la pression sera moins forte. Les efforts pourront être réduits entre 15 et 25 DaN. Si le montage était motorisé, on pourrait régler le couple du moteur afin de pouvoir obtenir une force idéale pour chaque patient.

Le pied 6 est susceptible d'une rotation latérale autour d'un axe 17, figure 1, orienté dans le sens du pied. Typiquement une telle rotation correspond à une prise de carre quand on fait du ski. De manière à ce que, de préférence, l'orthèse permette d'un tel débattement, tout en autorisant le relevage du pied comme on le verra plus loin, la tige 15 de vérin est munie, à l'endroit de sa fixation au sommet 11 de la tige de semelle 4, d'un cardan 18 autorisant une rotation de la semelle 4 autour d'une direction 17 perpendiculaire à la tige 15. Dans un exemple, figure 2, l'angle 19 de rotation autour de l'axe 17 peut être de l'ordre de plus ou moins 40°. A la place d'un cardan 18, bien entendu il est possible de monter un système à rotule, voire un système avec une liaison par une rotule de caoutchouc, autorisant un degré de liberté en rotation autour de l'axe 17, mais n'autorisant pas en lui-même de degrés de liberté selon le sens de la tige 15.

Le fonctionnement de l'orthèse de l'invention est le suivant. Son principe est lié à la présence de l'articulation 20 de la cheville qui sert de centre de rotation pour le relevage du pied. En pratique, l'effort du vérin 10 se réalise sur l'arrière de la semelle 4, à l'endroit du talon du pied 6. On suppose par exemple que le vérin 11 est en légère compression, par exemple parce que le pied est ballant et qu'il pèse sur la semelle 4. Le vérin 10 par sa tige 15 va donc avoir tendance à repousser l'extrémité 11 loin du bracelet 2. La semelle 4 a alors tendance à s'écarter du bracelet 2. Comme la semelle 4 est introduite dans une chaussure, la chaussure prenant appui sur le coup de pied 21 du pied 6, l'effort du vérin 10 ne peut pas conduire au désaccouplement de la semelle 4 de son appui sous la voûte plantaire du pied 6. Dans ces conditions, et dans la mesure où les ligaments de la cheville 20 empêchent la désarticulation du pied 6. L'effort du vérin 10 ne peut plus que contribuer au redressement de l'extrémité avant 22 du pied 6. Ainsi, un effort 23 exercé par le vérin 10 retentit en un redressement 24 du pied 6. S'il n'y a pas de chaussure, on peut par exemple prévoir des lanières ou sangles reliant la semelle 4 au pied 6 par le dessus des orteils et ou du coup de pied 21.

Bien entendu, lorsque le pied 6 est refoulé, par exemple lors de la marche lorsque le pied appareillé est dans la position arrière par rapport au marcheur, un phénomène inverse se produit. Si le pied 6 est refoulé, la tige 15 est sortie du corps 14 du vérin 10 au-delà de la position d'équilibre. L'effort exercé par cette tige 15, au-delà de sa position d'équilibre, est alors un effort de traction de sens inverse à la direction 23, tendant aussi à redresser le pied 6 dans une direction inverse à la direction 24.

On constate qu'une telle mobilisation est favorable à la marche. En effet, au moment où le pied arrière appareillé passe sur le devant, le pied se redresse : ici l'effort est fourni par le vérin 10 qui passe d'une position en extension à sa position d'équilibre. Au moment de la marche, le pied appareillé est ainsi amené à quitter la position arrière pour passer à côté de l'autre jambe et passer sur le devant du marcheur. Au moment où il passe à côté de la jambe, le pied est relevé conduisant le marcheur à ne pas avoir à lever exagérément la jambe appareillée pour éviter les effets de pied tombant, dit de steppage, par lequel le pied butte partout. On note par ailleurs un effort, léger, de relance de la marche fourni alors par la rétractation du vérin.

Pour la réalisation du pas, le pied appareillé conduit ensuite à un appui talonnier puis au déroulé du pas. Cet appui talonnier comporte quasiment immédiatement, une abattée de l'avant du pied 22 pour que la voûte plantaire soit en appui à plat sur le sol. Au moment de cette abattée, la tige 15 est amenée à entrer dans le corps 14 du vérin 10, au-delà de la position d'équilibre, avec une légère résistance, contribuant ainsi à un effet d'amortissement du choc du pas.

Dans un perfectionnement, le vérin à gaz 10 peut être muni d'un amortisseur de façon à améliorer encore le comportement dynamique du relevage du pied. En effet, si le moyen 10 est seulement un ressort, on comprend qu'en soumettant la pointe du pied libre à un effort dans un sens, et en la lâchant, elle regagnerait sa position d'équilibre par des oscillations désagréables à supporter. Pour éviter ces oscillations, la réponse de l'amortisseur 10 sera de préférence une réponse méplate, sans suroscillation.

Le bracelet 2 est fixé à la jambe 3 par des sangles telles que 25 qui sont par exemple et de préférence de type auto-agrippante. Ces sangles 25 sont ainsi enfilées dans des passants 26 placés en position quasi-fixe, puis sont auto agrippées sur elles mêmes. La mise en place de l'orthèse par l'utilisateur est ainsi facilitée (fixation du bracelet 2 possible avec une seule main).

Cependant, il est possible que l'utilisateur ne règle pas correctement la hauteur de fixation du bracelet 2 sur la jambe 3. Cette hauteur conduirait alors à réaliser une position d'équilibre du pied 6, par rapport à la jambe 3, qui pourrait ne pas être perpendiculaire mais être par exemple légèrement déroulée ou légèrement refoulée. De manière à aider l'utilisateur à trouver la bonne hauteur de réglage, on prévoit, à titre de perfectionnement, de relier en outre la semelle 4 au bracelet 2 par une sangle 27 dont les extrémités sont de préférence fixées de part et d'autre du bracelet 2 (sur les parties intérieure et extérieure de la jambe). Cette sangle 27 peut de préférence coulisser dans la semelle 4 en utilisant une gaine noyée également entre les deux demi-coques 7 et 8, ce qui permet de garder une bonne supination et une bonne pronation.

On peut également utiliser deux sangles 27 et 28, placées latéralement, de préférence de part et d'autre, sur les côtés de la jambe 3 et du pied 6. Les longueurs des sangles sont préréglées aux longueurs idéales pour le patient. Les sangles 27 et 28 sont par exemple reliées à la tige 5 de la semelle 4 par des pattes respectivement 29 et 30 qui sont déployées entre les coques 7 et 8. A leurs autres extrémités, la sangle 27 ou les sangles 27 et 28 sont fixées au bracelet 2 par l'intermédiaire de fixations 31 à rotation libre. De telles rotations libres sont de nature à autoriser le basculement expliqué jusqu'ici du pied 6 autour de la cheville 20.

Pour tenir compte de la rotation latérale autour de l'axe 17, les sangles 27 et 28 peuvent être réalisées en un matériau légèrement extensible. Les sangles 27 et 28 n'étant par ailleurs pas indispensables, on peut prévoir qu'elles seront amovibles, notamment à l'endroit de la fixation 31 et à l'endroit des fixations sur les pattes 29 et 30. Le rôle des sangles 27 et 28 est ainsi de limiter la poussée du vérin 10, ou sa traction lorsqu'il est mal réglé, en hauteur notamment. La position des sangles passant au droit de la cheville 20 contrebalance ainsi l'effort du vérin 10.

En remplacement du vérin 10 on peut également monter deux vérins côte à côte tant par choix technologique que dans le cas d'une pathologie spécifique ou complexe pour aboutir au résultat recherché. De même, les sangles 27 et ou 28 pourraient être remplacées par des amortisseurs eux aussi de préférence à gaz ou par un système à ressort(s), hydropneumatique voire motorisé, mais également par un système permettant un blocage pour limiter une pronation ou une supination trop prononcée.

On constate que l'orthèse de l'invention autorise une légère rotation externe du pied au moment de la marche. Dans ce but, les ancrages 12 et 13 sont légèrement déportés sur l'arrière perpendiculairement à la direction de la jambe 3. De cette façon la structure peut légèrement se vriller par rapport à la direction de la jambe 10 pour autoriser la rotation alternative du pied 6 à chaque pas.

Enfin, l'orthèse peut être adaptée pour la rééducation fonctionnelle, pour la rééducation progressive de muscles ou groupes musculaires participant à la marche.

Elle est plus particulièrement adaptée aux articulations qui sont bloquées dans une attitude vicieuse déficiente, faute d'actions musculaires et qui par conséquent finiront par se synostoser (solidifier) ou s'arthroser si elles manquent de mobilité (exemple : plâtre au long cours), et permet de fonctionner dans des conditions normales d'activité. Puisque la production de liquide articulaire se fait par inhibition, il est donc impératif que les articulations bougent pour maintenir leur intégrité. L'orthèse de l'invention permet une telle mobilité.

## Revendications

1. Orthèse dynamique (1) comportant un bracelet (2) de jambe et une semelle (4) de pied (6) relies ensemble par une structure (10), la structure comportant un vérin élastique déporté (12, 13) sur l'arrière de la jambe et du pied, ladite structure étant extensible longitudinalement (14, 13) pour écarter une partie (9) de la semelle du bracelet, **caractérisé en ce qu'**elle comporte une chaussure habituelle et **en ce que** ladite semelle étant une semelle de voûte plantaire pour être introduite dans la chaussure.

2. Orthèse selon la revendication 1, **caractérisée en ce que** le vérin élastique longitudinal est un vérin pneumatique.

3. Orthèse selon la revendication 2, **caractérisée en ce que** le vérin comporte un moyen (16) pour régler sa pression.

4. Orthèse selon l'une des revendications 2 à 3, **caractérisée en ce que** le vérin comporte un état d'équilibre, sans poussée ou sans traction supérieure à la force musculaire, pour garder une position du pied perpendiculaire à la jambe.

5. Orthèse selon l'une des revendications 1 à 4, **caractérisée en ce que** le vérin élastique comporte un moyen (18) pour permettre une rotation de la semelle selon un axe (17) horizontal orienté dans le sens du pied.

6. Orthèse selon la revendication 5, **caractérisée en ce que** le moyen pour permettre une rotation comporte un cardan ou une rotule ou une liaison élastique dont le lieu de rotation est coaxial (17, 20) à l'axe de rotation latérale d'un pied d'un utilisateur de l'orthèse.

7. Orthèse selon l'une des revendications 1 à 6, **caractérisée en ce que** la structure prend appui sur l'arrière (11) de la semelle de façon à relever (24) l'avant du pied lorsque le pied est libre de s'affaisser.

8. Orthèse selon l'une des revendications 1 à 7, **caractérisée en ce que** la structure comporte une sangle (27) reliée au bracelet et à la semelle, sur au moins un bord latéral de la jambe, pour limiter la poussée ou la traction du vérin.

9. Orthèse selon la revendications 8, **caractérisée en ce que** la sangle comporte une fixation (31) sur le bracelet qui est à rotation libre.

10. Orthèse selon l'une des revendications 1 à 9, **caractérisée en ce que** le bracelet comporte des sangles (25) auto-agrippantes (26).

11. Orthèse selon l'une des revendications 1 à 10, **caractérisée en ce que** la semelle comporte une tige (5) rigide, et une coque (7, 8) anatomique, par exemple en fibre de verre, moulée sur le pied d'un patient et surmoulée sur cette tige.

12. Orthèse selon la revendication 11, **caractérisée en ce que** la semelle a une forme anatomique surmoulée des deux côtés.

## Claims

1. A dynamic orthosis (1) comprising a leg holder (2) and a foot (6) plate (4) connected together by a structure (10), the structure comprising an elastic cylinder (12, 13) shifted to the back of the leg and the foot, said structure being longitudinally extensible (14, 13) for moving a part (9) of the foot plate from the leg holder, **characterized in that** the orthosis comprises a customary shoe and **in that** said foot plate is a plate for the arch of the foot to be introduced in the shoe.

2. The orthosis according to claim 1, **characterized in that** the longitudinal elastic cylinder is a pneumatic cylinder.

3. The orthosis according to claim 2, **characterized in that** the cylinder comprises a means (16) for adjusting its pressure.

4. The orthosis according to one of claims 2 to 3, **characterized in that** the cylinder comprises a state of equilibrium, without thrust or force greater than muscular force, to maintain a position of the foot that is perpendicular to the leg.

5. The orthosis according to one of claims 1 to 4, **characterized in that** the elastic cylinder comprises a means (18) for allowing a rotation of the foot plate according to a horizontal axis (17) oriented in the direction of the foot.

6. The orthosis according to claim 5, **characterized in that** the means for allowing a rotation comprise a universal joint or a swivel joint or an elastic connection in which the locus of rotation is coaxial (17, 20) to the lateral axis of rotation of a foot of an orthosis user.

7. The orthosis according to one of claims 1 to 6, **characterized in that** the structure is supported by the back (11) of the foot plate in such a way as to raise (24) the front of the foot when the foot is free to fall.

8. The orthosis according to one of claims 1 to 7, **characterized in that** the structure comprises a strap (27) connected to the leg holder and to the foot plate, on at least one lateral edge of the leg, to limit the force or the thrust of the cylinder.

9. The orthosis according to claim 8, **characterized in that** the strap comprises a fastening (31) on the leg holder that is at free rotation.

10. The orthosis according to one of claims 1 to 9, **characterized in that** the leg holder comprises self-adhesive (26) fastenings (25).

11. The orthosis according to one of claims 1 to 10, **characterized in that** the foot plate comprises a rigid rod (5), and an anatomically designed body (7, 8), for example in fiberglass, molded on the foot of a patient and overmolded onto this rod.

12. The orthosis according to claim 11, **characterized in that** the foot plate has an anatomically designed shape that is overmolded on both sides.

## Patentansprüche

1. Dynamische Orthese (1), die ein Beinband (2) und eine Sohle (4) des Fußes (6) umfasst, die miteinander durch eine Struktur (10) verbunden sind, wobei die Struktur einen auf die Rückseite des Beins und des Fußes verlagerten elastischen Zylinder (12, 13) umfasst, wobei die Struktur längs (14, 13) verlängerbar ist, um einen Teil (9) der Sohle von dem Band zu beabstanden, **dadurch gekennzeichnet, dass** sie einen gewöhnlichen Schuh umfasst und dadurch, dass die Sohle eine Fußgewölbesohle ist, die in den Schuh eingefügt wird.

2. Orthese nach Anspruch 1, **dadurch gekennzeichnet, dass** der elastische Längszylinder ein Druckluftzylinder ist.

3. Orthese nach Anspruch 2, **dadurch gekennzeichnet, dass** der Zylinder ein Mittel (16) umfasst, um seinen Druck einzustellen.

4. Orthese nach einem der Ansprüche 2 bis 3, **dadurch gekennzeichnet, dass** der Zylinder einen Gleichgewichtszustand ohne Schub oder ohne Zug größer als die Muskelkraft umfasst, um eine Stellung des Fußes senkrecht zum Bein zu wahren.

5. Orthese nach einem der Ansprüche 1 bis 4, **dadurch gekennzeichnet, dass** der elastische Zylinder ein Mittel (18) umfasst, um eine Drehung der Sohle gemäß einer Achse (17) horizontal in die Richtung des Fußes orientiert zu erlauben.

6. Orthese nach Anspruch 5, **dadurch gekennzeichnet, dass** das Mittel zum Erlauben einer Drehung ein Kardangelenk oder ein Kugelgelenk oder eine elastische Verbindung umfasst, dessen Drehstelle (17, 20) zur seitlichen Rotationsachse eines Fußes eines Orthesebenutzers koaxial ist.

7. Orthese nach einem der Ansprüche 1 bis 6, **dadurch gekennzeichnet, dass** die Struktur auf der Rückseite (11) der Sohle so aufliegt, dass die Vorderseite des Fußes angehoben (24) wird, wenn der Fuß frei ist, um nachzugeben.

8. Orthese nach einem der Ansprüche 1 bis 7, **dadurch gekennzeichnet, dass** die Struktur einen Gurt (27) umfasst, der mit dem Band und der Sohle auf mindestens einem seitlichen Rand des Beins verbunden ist, um den Schub oder den Zug des Zylinders einzuschränken.

9. Orthese nach Anspruch 8, **dadurch gekennzeichnet, dass** der Gurt eine Befestigung (31) auf dem Band umfasst, die in freier Drehung ist.

10. Orthese nach einem der Ansprüche 1 bis 9, **dadurch gekennzeichnet, dass** das Band selbsthaftende (26) Gurte (25) umfasst.

11. Orthese nach einem der Ansprüche 1 bis 10, **dadurch gekennzeichnet, dass** die Sohle einen starren Schaft (5) und eine anatomischen Schale (7, 8) zum Beispiel aus Glasfasern umfasst, die auf den Fuß eines Patienten geformt und auf diesen Schaft abgeformt wird.

12. Orthese nach Anspruch 11, **dadurch gekennzeichnet, dass** die Sohle eine auf beiden Seiten abgeformte anatomische Form hat.
